# EUROPEAN PATENT SPECIFICATION

(11) **EP 4 642 388 B1**
(45) Date of publication and mention of the grant of the patent: **21.10.2026**
(21) Application number: 24709595.3
(22) Date of filing: 29.01.2024
(51) Int. Cl.: A61F 2/24, B33Y 40/20

(54) **FERROMAGNETIC SKIRT FOR A MEDICAL DEVICE**
FERROMAGNETISCHER SOCKEL FÜR EINE MEDIZINISCHE VORRICHTUNG
JUPE FERROMAGNÉTIQUE POUR DISPOSITIF MÉDICAL

(30) Priority: 30.01.2023 US 202363482182 P
(43) Date of publication of application: 05.11.2025
(73) Proprietor: Edwards Lifesciences Corporation, Irvine, CA 92614 (US)
(72) Inventor: RAY, Laxmi, Costa Mesa, California 92627 (US); ROSS, Julia, Leigh, Huntington Beach, California 92649 (US); AXELROD, Blake, W., Irvine, California 92614 (US)
(74) Representative: Venner, Julia Ann
(86) International application number: PCT/US2024/013412
(87) International publication number: WO 2024/163373

(56) References cited:
- US-A1- 2014 214 149
- US-A1- 2016 157 868

## Description

### CROSS-REFERENCE TO RELATED APPLICATION(S)

This application claims the benefit of U.S. Provisional Application No. 63/482,182, filed January 30, 2023, and entitled "FERROMAGNETIC SKIRT FOR A MEDICAL DEVICE," .

### BACKGROUND

The present disclosure relates to implantable medical devices, and in particular, to implantable prosthetic valves.

Implantable medical devices, including artificial heart valves, include sensors with antennae to transmit information measured by the sensors, for example to an external device positioned outside of the body. For example, an inductive coil antenna and sensors can be integrated onto a metal heart valve frame. However, proximity of the inductive coil to the metal frame causes the metal frame to create eddy currents which cause a secondary magnetic field in phase opposition to the primary field. This results in detuning effects in the inductive coil including shifting a resonance frequency of the inductive coil and limiting a wireless detection range of sensors.

US 2014/214149 describes a prosthesis for monitoring a characteristic of flow which includes a first tubular prosthesis having a lumen and a sensor for detecting the characteristic of flow through the lumen. The sensor may be covered with another tubular prosthesis or by a layer of material in order to insulate the sensor from the fluid flow. A pocket may be formed between the tubular prosthesis and the adjacent layer of material or prosthesis and the sensor may be disposed in the pocket.

### SUMMARY

An aspect of the presently claimed invention is set out in the independent claim. Particular embodiments of this aspect are set out in the dependent claims. Any subject matter contained herein that does not fall within the scope of the appended claims is considered as being useful for understanding the invention

An implantable medical device includes a wire frame, a cover, and a sensor. The wire frame is formed of struts and openings. The cover connects to the struts of the wire frame. The cover is fashioned from a fabric including a PET fabric made of PET yarn and a ferromagnetic material combined with the PET fabric. The PET yarn is made from a plurality of PET fibers. The sensor is positioned on the cover and connected to the wire frame. The cover shields the sensor from detuning effects of the wire frame.

### BRIEF DESCRIPTION OF THE DRAWINGS

FIG. 1 is a partial cross-sectional schematic of a human heart with the four chambers shown in cross-section.
FIG. 2 is a block diagram representing a monitoring system for monitoring one or more physiological parameters associated with a patient.
FIG. 3 is an isometric view of a prosthetic heart valve.
FIG. 4 is a block diagram representing select components of an active sensing circuit of the prosthetic heart valve.
FIG. 5 is a schematic cross-sectional view of a portion of the prosthetic heart valve of FIG. 3.
FIG. 6 is an isometric view of the prosthetic heart valve covered by a ferromagnetic skirt.
FIG. 7 is a schematic cross-sectional view of a first example of a fabric
FIG. 8 is a schematic cross-sectional view of a second example of a fabric.
FIG. 9A is a cross-sectional view of ferromagnetic yarn and PET yarn.
FIG. 9B is a cross-sectional view of a spun ferromagnetic and PET yarn.
FIG. 9C is a cross-sectional view of a coated PET yarn.
FIG. 10 is a schematic view of a woven fabric used to form the ferromagnetic skirt.
FIG. 11 is a schematic view of a braided fabric used to form the ferromagnetic skirt.
FIG. 12 is a schematic view of a knit fabric used to form the ferromagnetic skirt.
FIG. 13 is a schematic view of a non-woven fabric used to form the ferromagnetic skirt.

### DETAILED DESCRIPTION

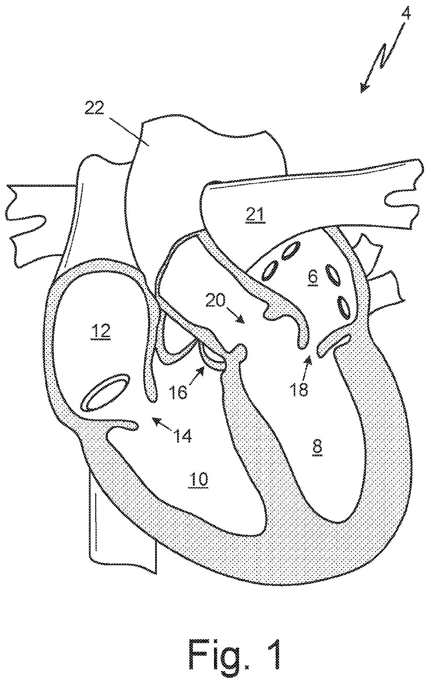

FIG. 1 is a partial cross-sectional schematic of heart 4. Heart 4 includes four chambers, including left atrium 6, left ventricle 8, right ventricle 10, and right atrium 12. The four chambers are shown in cross-section in FIG. 1. Heart 4 further includes four valves for aiding the circulation of blood therein, including tricuspid valve 14, pulmonary valve 16, mitral valve 18, and aortic valve 20. FIG. 1 further shows pulmonary artery 21 and artery 22.

Tricuspid valve 14 separates right atrium 12 from right ventricle 10 and can include three cusps or leaflets. Tricuspid valve 14 can close during ventricular contraction (i.e., systole) and open during ventricular expansion (i.e., diastole). Pulmonary valve 16 separates right ventricle 10 from pulmonary artery 21 and may be configured to open during systole so that blood may be pumped towards the lungs, and close during diastole to prevent blood from leaking back into heart 4 from pulmonary artery 21. Similar to tricuspid valve 14, pulmonary valve 16 can have three cusps/leaflets, each one resembling a crescent. Mitral valve 18 separates left atrium 6 from left ventricle 8 and can have two cusps or leaflets. Mitral valve 18 is configured to open during diastole so that blood in left atrium 6 can flow into left ventricle 8, and close during systole to prevent blood from leaking back into left atrium 6. Aortic valve 20 separates left ventricle 8 from aorta 22. Aortic valve 20 is configured to open during systole to allow blood leaving left ventricle 8 to enter aorta 22, and close during diastole to prevent blood from leaking back into left ventricle 8.

A heart valve can include a relatively dense fibrous ring, referred to herein as the annulus, as well as a plurality of leaflets or cusps attached to the annulus. Some valves can further include a collection of chordae tendineae and papillary muscles securing the leaflets. Generally, the size of the leaflets or cusps may be such that when the heart contracts, the resulting increased blood pressure produced within the corresponding heart chamber forces the leaflets open at least partially to allow flow from the heart chamber. As the pressure in the heart chamber subsides, the pressure in the subsequent chamber or blood vessel may become dominant and press back against the leaflets. As a result, the leaflets/cusps come in apposition to each other, thereby closing the flow passage.

Heart valve disease represents a condition in which one or more of the valves of heart 4 fails to function properly. Diseased heart valves may be categorized as stenotic, wherein the valve does not open sufficiently to allow adequate forward flow of blood through the valve, and/or incompetent, wherein the valve does not close completely, causing excessive backward flow of blood through the valve when the valve is closed. In certain conditions, valve disease can be severely debilitating and even fatal if left untreated.

To treat disease of, for example, mitral valve 18, a prosthetic heart valve can be implanted in and sutured to the annulus of mitral valve 18. Such a prosthetic heart valve can be positioned with its openings oriented in the direction of blood flow from left atrium 6 to left ventricle 8. The prosthetic heart valve can be configured to operate as aortic valve 20 such that it can allow unidirectional blood flow to left ventricle 8 from left atrium 6 while preventing flow in the reverse direction.

In a typical cardiac implant procedure, the heart can be incised, and in a valve replacement operation, the defective valve can be removed leaving the desired placement site that can include the valve annulus. Sutures can be passed through fibrous tissue of the annulus or desired placement site to form an array of sutures. Free ends of the sutures may be individually threaded through a suture-permeable sealing edge of the prosthetic heart valve. Artificial heart valves can be used to replace faulty or deteriorating natural heart valves in patients with heart valve disorders including aortic stenosis, mitral regurgitation, etc. The valve replacement process generally involves surgical or transcatheter procedures (e.g., balloon valvotomy) to replace the existing valves with the new artificial valves. Since the artificial valves are a foreign body, many different challenges and issues can be involved with such a procedure. For example, paravalvular leakage (PVL) and/or leaflet thickening can occur in patients who undergo heart valve replacement. Similarly, rejection of an artificial surgical heart valve due to thrombus can occur, requiring the patient to use anti-coagulants for proper valve operation.

Some methods for monitoring valve performance after implantation involve using complex bio-imaging techniques, such as echocardiography. Such methods can generally only be performed in specialized medical facilities and can cost significant time and money. Hence, such methods may generally only be used once symptoms of valve malfunction are detected. Some artificial valves may not provide an ability to detect changes in operation to detect problems early on. Moreover, many patients who suffer from valvular disease and require an artificial valve may also suffer from other cardiovascular disorders, including heart failure. Some artificial heart valve systems may not allow for gathering data about the valve and/or the patient's condition postoperatively in an outpatient setting (e.g., a cardiologist visit in a ward) using existing patient monitoring systems. Such systems may not provide for routine collection of data at sufficient resolution to enable development of new digital solutions for better management of the patients as their numbers and diversity increase over time.

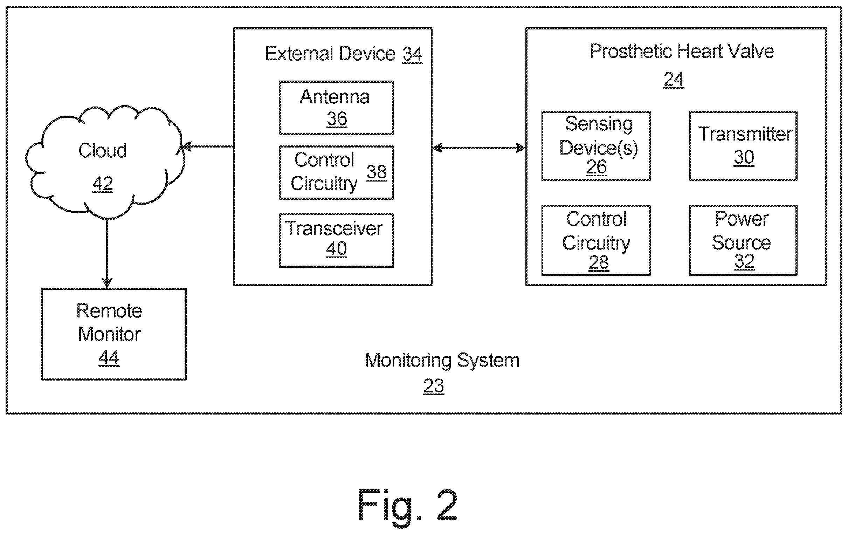

Accordingly, a prosthetic heart valve can be part of a larger system for postoperatively monitoring a patient, as will be discussed in reference to FIG. 2.

FIG. 2 is a block diagram representing monitoring system 23 for monitoring one or more physiological parameters associated with a patient. System 23 includes prosthetic heart valve 24, which includes sensing devices 26, control circuity 28, transmitter 30, and power source 32. System 23 further includes external device 34, which includes antenna 36, control circuity 38, and transceiver 40. System 23 also includes cloud 42 and remote monitor 44.

Prosthetic heart valve 24 can include one or more sensing devices 26, control circuitry 28, transmitter 30, and power source 32. Sensing devices 28 can include one or more of following types of sensors/transducers: microelectromechanical system (MEMS) sensors, optical sensors, piezoelectric sensors, electromagnetic sensors, strain sensors/gauges, accelerometers, gyroscopes, and/or other types of sensors, which can be positioned in the patient to sense one or more parameters relevant to the health of the patient. Control circuitry 28 can be wired or wirelessly connected to sensing devices 26 and can include one or more of application-specific integrated circuit (ASIC), microcontrollers, chips, tuning capacitors, etc. Control circuitry 28 can receive signals from external device 34 (e.g., requests for stored or immediately acquired data), request data from sensors 26, and coordinate data transmission. Transmitter 30 can be, for example, an antenna for radiating an electronic signal transmitted by control circuitry 28. Power source 32 can be a suitable source of power able to minimize interference with the heart or other anatomy of the patient. In one example, power source 32 can be a passive means for wirelessly receiving external power (e.g., short-range or near-field wireless power transmission). In another example, power source 32 can be a battery, or a means for locally harvesting energy from within the patient.

External device 34, located at least partially outside of the patient, can be in wireless communication with prosthetic heart valve 24. External device 34 includes antenna 36, control circuitry 38, and transceiver 40. Antenna 36 can receive wireless signal transmissions from prosthetic heart valve 24. In one example, antenna 36 can be externally mounted to external device 34. Control circuitry 38 can be a processor or other suitable means for processing signals received from prosthetic heart valve 24. Transceiver 40 can be configured to receive and amplify signals from prosthetic heart valve 24, as well as to transmit signals to cloud 42 and remote monitor 44. Such signals can include, for example, pressure data acquired from sensors 26. Transceiver 40 can, accordingly, include one or more digital-to-analog converter (DAC) circuitry, power amplifiers, low-pass filters, antenna switch modules, antennas, etc. for treatment and/or processing of transmitted and received signals.

External device 34 can serve as an intermediate communication device between prosthetic heart valve 24 and remote monitor 44. External device 34 can be a dedicated external unit designed to communicate with prosthetic heart valve 24. For example, external device 34 can be a wearable communication device, or other device that can be readily disposed in proximity to the patient and/or prosthetic heart valve 24. External device 34 can be configured to interrogate prosthetic heart valve continuously, periodically, or sporadically 24 in order to extract or request sensor-based information therefrom. In some examples, external device 34 can include a user interface upon which a user (e.g., the patient) can view sensor data, request sensor data, or otherwise interact with external device 34 and/or prosthetic heart valve 24.

Cloud 42 can be a secure network in communication with external device 34 via ethernet, Wi-Fi, or other network protocol. Cloud 42 can also be configured to implement data storage. In another example, cloud 42 can instead be a secure physical network. Remote monitor 44 can be in communication with external device 34 via cloud 42. Remote monitor 44 can be any type of computing device or collection of computing devices configured to receive, process and/or present monitor data received via cloud 42 from external device 34 or prosthetic heart valve 24. For example, remote monitor 44 can advantageously be operated and/or controlled by a healthcare entity, such as a hospital, doctor, or other care entity associated with the patient. Although certain examples disclosed herein describe communication with remote monitor 44 from prosthetic heart valve 24 indirectly through external device 34, prosthetic heart valve 24 can instead include a transmitter (e.g., transmitter 30) capable of communicating, via cloud 42, with remote monitor 44 without the necessity of relaying information through device 34.

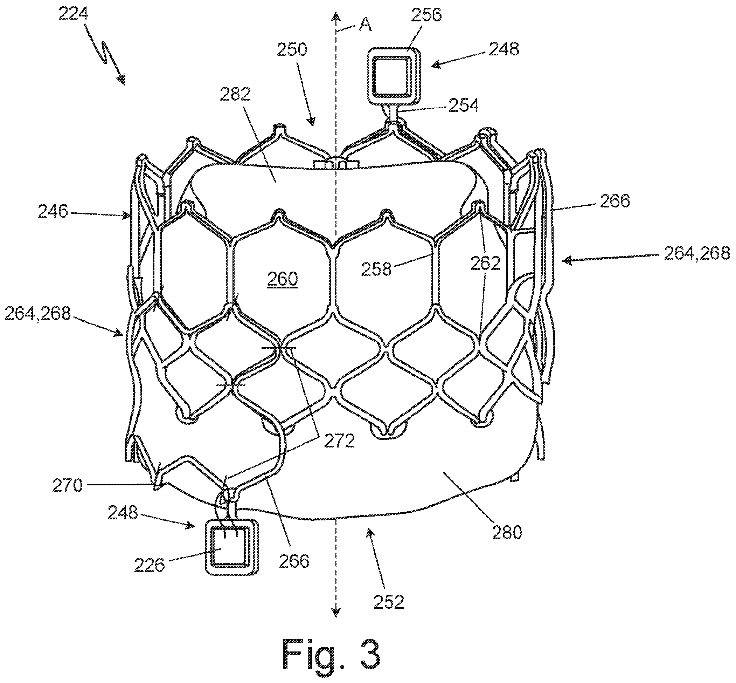

FIG. 3 is an isometric view of prosthetic heart valve 224. Heart valve 224 includes sensors 226, deformable frame 246, post assemblies 248, upper end 250, lower end 252, posts 254, and islets 256. Frame 246 also includes struts 258, cells 260, and tips (or ends) 262. Heart valve 224 also includes sensing circuits 264. In FIG. 3, sensing circuits 264 include antenna coils 266, circuits 268, and pointed tips 270. Heart valve 246 also includes sutures 272, skirt 280, and pericardium tissue 282.

FIG. 3 shows structural components of prosthetic heart valve 224, which include deformable frame 246 and post assemblies 248 extending axially away from frame 246 relative to valve axis A. Axis A can generally be aligned with the direction of blood flow through prosthetic heart valve 224 when implanted. Frame 246 can be formed from a biocompatible metallic material. Frame 246 can be formed of wire made of a biocompatible metallic material. As shown in FIG. 3, one post assembly 248 extends from each of top/upper end 250 and bottom/lower end 252 of prosthetic heart valve 224 based on the orientation of FIG. 3. Each post assembly 248 can include post 254 and islet 256 upon which sensor 226 can be mounted. As shown in FIG. 3, islet 256 can have a generally square shape corresponding to the shape of sensor 226. Frame 246 comprises a network of struts 258 defining open cells 260 therebetween. Each cell 260 can include oppositely axially disposed pointed tips/ends 262.

Electrical components of prosthetic heart valve 224 include one or more sensing circuits 264 for monitoring physiological parameters of a patient with prosthetic heart valve 224. Sensing circuit 264 includes deformable antenna coil 266 and sensor 226 electrically connected (e.g., via leads/wires) to antenna coil 266. Antenna coil 266 can also be referred to as an inductor coil.

In one example, shown in FIG. 3, sensing circuit 264 is an inductor-resistor-capacitor (LCR) circuit 268, with antenna coil 266 forming the inductor (L) and resistor (R) elements of circuit 268, and sensor 226, connected in parallel, forming the capacitor (C) element. Each LCR circuit 268 of prosthetic heart valve 226 has a distinct self-resonant frequency. The self-resonant frequency for each circuit can be represented as *f =* 1/2*π*√*LC*(*p*), where L is the inductance of antenna coil 266 and C(p) is the capacitance of sensor 226 at a given pressure. In general, the self-resonant frequency for each LCR circuit 268 can range from 5 MHz to 50 MHz, and more specifically, from 10 MHz to 20 MHz.

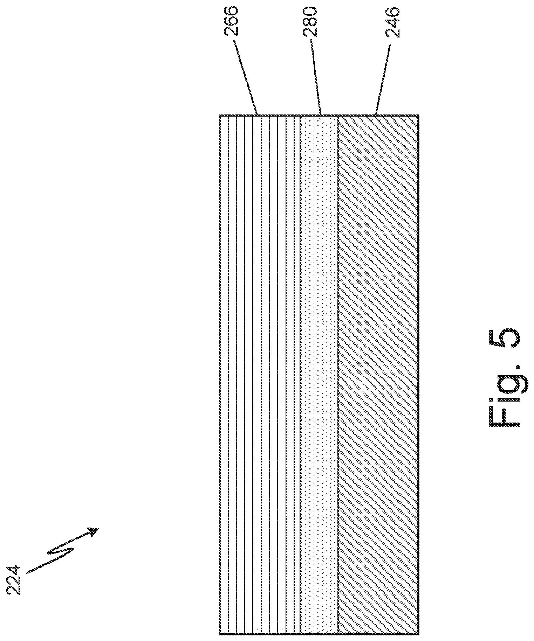

Antenna coil 266 can include one or more individual wires formed from a conductive, but biocompatible, metallic material, such as gold. Other examples can include copper or titanium. Antenna coil 266 can further be coated with an insulating coating. Sensors 226 can be capacitive pressure sensors in one example, each including a diaphragm and pressure cavity to form a variable capacitor to detect strain due to pressure applied to the diaphragm. In general, the capacitance of sensors 226 decreases as pressure deforms the diaphragms. To manage detuning of sensing circuit 264, a detuning mitigation layer, discussed in greater detail below with respect to FIGS. 5-11, can be positioned between antenna coil 266 and struts 258 of frame 246.

Antenna coil 266 can be removably attached to frame 246 by sutures 272, shown in FIG. 3. Sutures 272 can be formed from a biocompatible polymer, ferromagnetic material, radiopaque material, and combinations thereof. More specifically, antenna coil 266 can be attached to frame 246 in such a manner as to trace a subset of struts 258 and outline a subset of cells 260. In this regard, antenna coil 266 can have nearly identical geometric attributes to struts 258 and cells 260, for example, having pointed tips 270 corresponding to pointed tips 262 of the underlying cells 260 of frame 246. In the example of FIG. 3, antenna coil 266 can be disposed to trace/frame/outline a two by three subset (i.e., two cells high in the axial direction and three cells long in a radial dimension) of cells 260 of frame 246. This can include uppermost or lowermost cells, along with interior cells 260. Other arrangements are contemplated herein. Sutures 272can be disposed at various points along antenna coil 266 to ensure that antenna coil 266 is secured to and maintains the shape of the supporting subset of struts 258. Suture points can include pointed tips 262 of cells 260 and of frame 246 and pointed tips 270 of antenna coil 266, respectively. Additional and/or alternative suture points are contemplated herein.

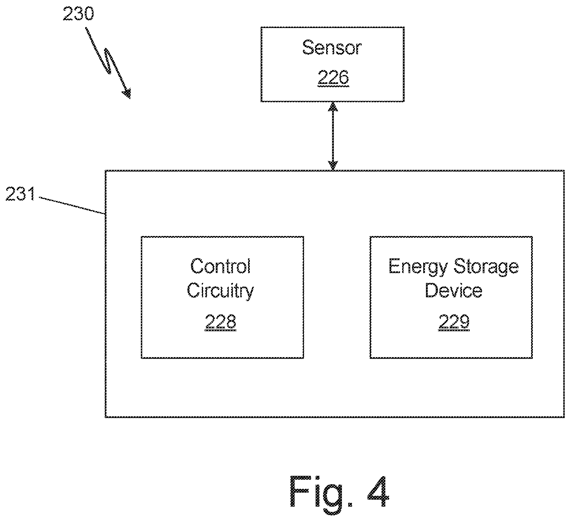

Prosthetic heart valve 224 includes skirt 280 which partially covers frame 246. Skirt 280 is fashioned from a biocompatible fabric. For example, skirt 280 can be formed from a polymer material. In an alternative example, skirt 280 can fully cover frame 246 such that no struts 258 are exposed on the outer side of frame 246. As will be discussed below in relation to FIGS. 4-13, skirt 280 can be made in part from a ferromagnetic material to reduce the generation of eddy currents by frame 246 when in contact with antenna coil 266. A combination of ferromagnetic material and radiopaque material can also be used to make skirt 280. Prosthetic heart valve 224 also includes pericardium tissue 282 which can be formed from a synthetic material or derived from a mammalian (e.g., bovine) tissue source. Pericardium tissue 282 forms valvular members that are held in frame 246 and open and close to allow blood to flow through prosthetic heart valve 224.

FIG. 4 is a block diagram representing select components of active sensing circuit 230, which can be associated with prosthetic heart valve 224. Active sensing circuit 230 includes control circuitry 228, energy storage device 229, and container 231. FIG. 4 also shows sensor 226.

Active sensing circuit 230 is a second example of antenna coil 266 (shown in FIG. 3). Active sensing circuit 230 is an alternative to LCR circuit 268 (shown in FIG. 3). In the second example, sensor 226 can be incorporated into active sensing circuit 230, as shown schematically in FIG. 4. More specifically, sensor 226 can be in communication with control circuitry 228 and energy storage device 229 (e.g., a capacitor or battery). Control circuitry 228 and energy storage device 229 can be housed in container 231, which can be formed from a biocompatible material and hermetically sealed to prevent exposure to surrounding tissue. Sensor 226 can be closely associated with container 231 (e.g., as a deformable membrane) but need not be sealed inside to permit probing of the external environment. For such active sensing applications, the self-resonant frequency can similarly range from 5 MHz to 50 MHz, and more specifically, from 10 MHz to 20 MHz. Active sensing circuit 230 can be positioned opposite skirt 280 from wire frame 246 of prosthetic heart valve 224 to allow for shielding.

FIG. 5 is a schematic cross-sectional view of a portion of prosthetic heart valve 224 shown in FIG. 3. FIG. 5 includes wire frame 246, antenna coil 266, and skirt 280.

Skirt 280 is placed between antenna coil 266 and wire frame 246. When antenna coil 266 is integrated onto wire frame 246, eddy currents are produced by the metal of wire frame 246. These eddy currents from wire frame 246 create a detuning effect on antenna coil 266. The detuning effect reduces wireless sensing capabilities by creating a tuned resonant frequency shift of antenna coil 266 and reducing the wireless sensing range of sensing devices (for example, sensing device(s) 26 shown in FIG. 2 or sensors 226 shown in FIG. 3).

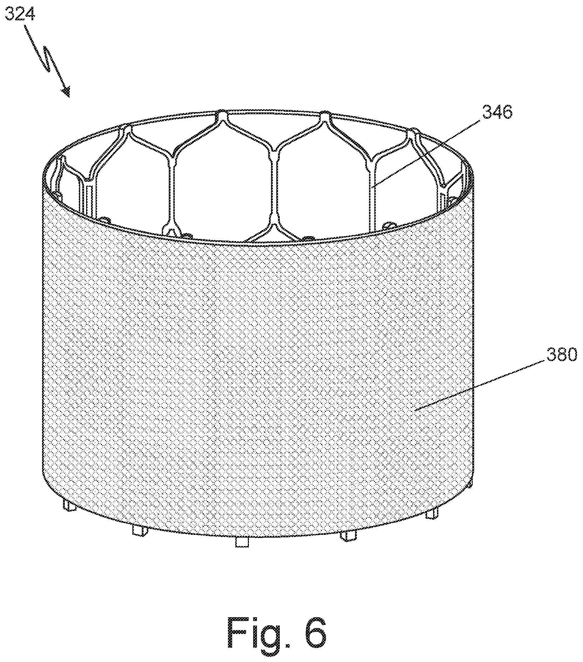

Skirt 280 is made from a fabric that includes a biocompatible material and a ferromagnetic material. Combining a ferromagnetic material, for example, zinc ferrite or manganese ferrite, with biocompatible materials to create a fabric used for skirt 280 shields wire frame 246 from antenna coil 266, suppresses eddy current formation, and reduces the detuning effects. Skirt 280 with integrated ferromagnetic materials also increases the signal strength of antenna coil 266 because the ferromagnetic material will act like a magnetic flux multiplier forming stronger near field inductive coupling between an external receiver (for example, external device 34 in FIG. 2) and the implantable sensing devices (for example, sensing devices 26 of FIG. 2). In air, a sensor range can double when skirt 280 includes ferromagnetic material. Example fabrics that skirt 280 can be fashioned from integrate soft ferromagnetic materials, biocompatible plastics, and/or radiopaque materials. Addition of radiopaque materials (for example, barium sulfate) can help reduce detuning effects. Example fabrics used to fashion skirt 280 will be discussed in relation to FIGS. 6-13 below.

FIG. 6 is an isometric view of prosthetic heart valve 324 with wire frame 346 covered by ferromagnetic skirt 380. Ferromagnetic skirt 380 extends from a top to a bottom of wire frame 346. Ferromagnetic skirt 380 is approximately 23 millimeters in length to cover wire frame 346. Configuring ferromagnetic skirt 380 as such allows for an inductor coil (for example, antenna coil 266 of FIG. 3) to be positioned anywhere on wire frame 346.

Fabrics used for ferromagnetic skirt 380 are flexible and can move with wire frame 346 from an expanded state (shown in FIG. 6) to a crimped state. For example, ferromagnetic skirt 380 should crimp with wire frame 346 to fit into a catheter for deployment of prosthetic heart valve 324. Also, ferromagnetic skirt 380 should expand with wire frame 346 to be deployed and positioned in a heart. Such ferromagnetic materials include, for example, zinc ferrite and manganese ferrite.

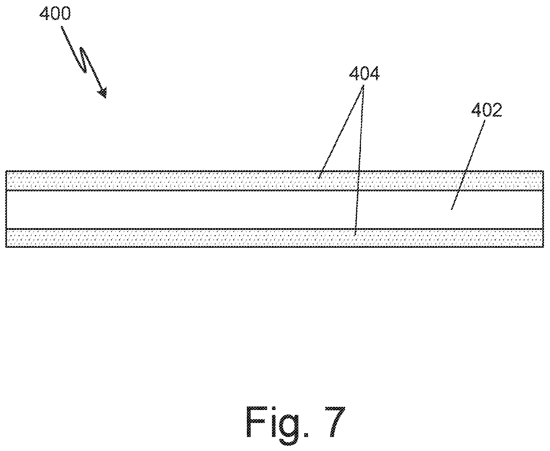

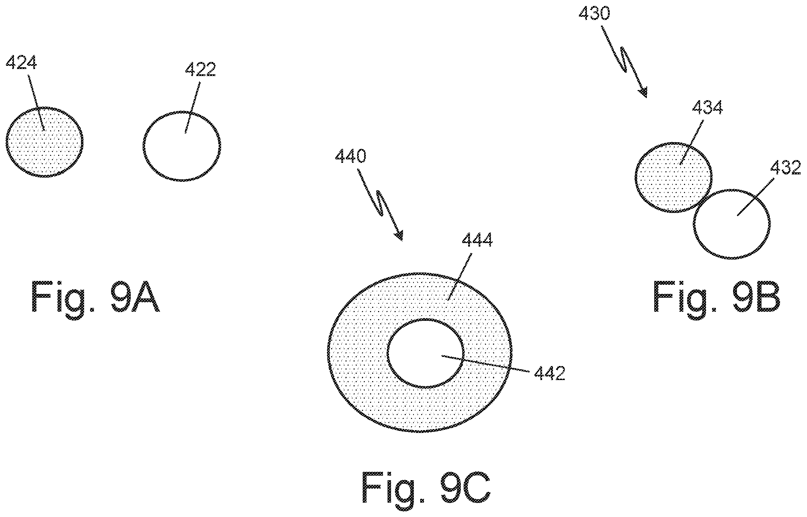

FIG. 7 is a schematic cross-sectional view of fabric 400, which includes PET fabric 402 and ferromagnetic material 404. Fabric 400 can be used to fashion skirt 280 (shown in FIGS. 3-5), skirt 380 (shown in FIG. 6), or any other skirt for a prosthetic valve device. PET fabric 402 is a middle layer of fabric 400 and is layered on either side with ferromagnetic material 404. Alternatively, ferromagnetic material 404 can be on only one side of PET fabric 402. PET fabric 402 is made from PET yarn using various methods of weaving, knitting, braiding, or electrospinning (discussed in relation to FIGS. 9A-13 below). Ferromagnetic material 404 is a coating onto PET fabric 402. Adding ferromagnetic material 404 on either side of PET fabric 402 gives fabric 400 the shielding properties required to suppress eddy current formation in a prosthetic heart valve with a wire frame and an inductor coil (for example prosthetic heart valve 224 in FIGS. 3-4), thereby reducing detuning effects, as discussed above in relation to FIGS. 3-4. PET fabric 402 makes fabric 400 flexible.

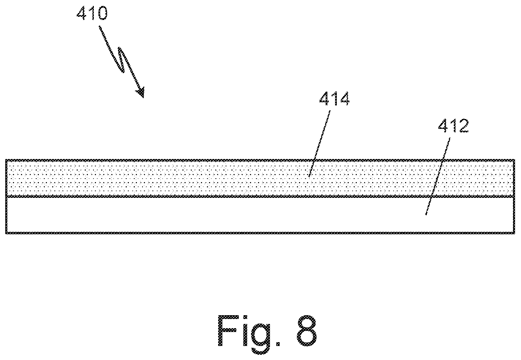

FIG. 8 is a schematic cross-sectional view of fabric 410, which includes PET fabric 412 and ferromagnetic fabric 414. Fabric 410 is constructed by layering PET fabric 412 and ferromagnetic fabric 414 together. PET fabric 412 and ferromagnetic fabric 414 can be adhered together to create fabric 410. Alternatively, PET fabric 412 and ferromagnetic fabric 414 can be stitched together using biocompatible sutures. PET fabric 412 and ferromagnetic fabric 414 can be stitched together when fabric 410 is being stitched to a prosthetic heart valve frame (for example, frame 246). A set of sutures can be used to simultaneously stitch PET fabric 412, ferromagnetic fabric 414, and the prosthetic heart valve frame together. Fabric 410 is attached to the prosthetic heart valve frame so that PET fabric 412 is adjacent to the frame and ferromagnetic fabric 414 is away from the frame.

FIGS. 7-8 are two examples of fabrics with layers that can be used to fashion skirt 280 of FIGS. 3-5 or skirt 380 of FIG. 6. A third example fabric that can be used for skirts 280 or 380 does not have layers made of different materials but is a single fabric with different materials integrated into the fabric. For example, a fabric woven with yarn with PET fibers and ferromagnetic fibers. More examples of a single fabric made from different materials will be discussed in relation to FIGS. 9A-13 below.

FIG. 9A is a cross-sectional view of PET yarn 422 and ferromagnetic yarn 424. FIG. 9B is a cross-sectional view of combined yarn 430. FIG. 9C is a cross-sectional view of a coated yarn 440. FIGS 9A-9C will be discussed together. Yarns 422, 424, 430, and 440 are yarns that can be combined to create ferromagnetic fabrics used to fashion a skirt for an implantable medical device (for example, skirt 280 of implantable medical device 224 or skirt 380 of implantable medical device 324). Using yarns 422, 424, 430, and 440 creates a fabric with ferromagnetic properties that can reduce detuning effects of a metal frame (for example, wire frame 246) on an inductor coil (for example, antenna coil 266), as discussed in relation to FIGS. 3-4.

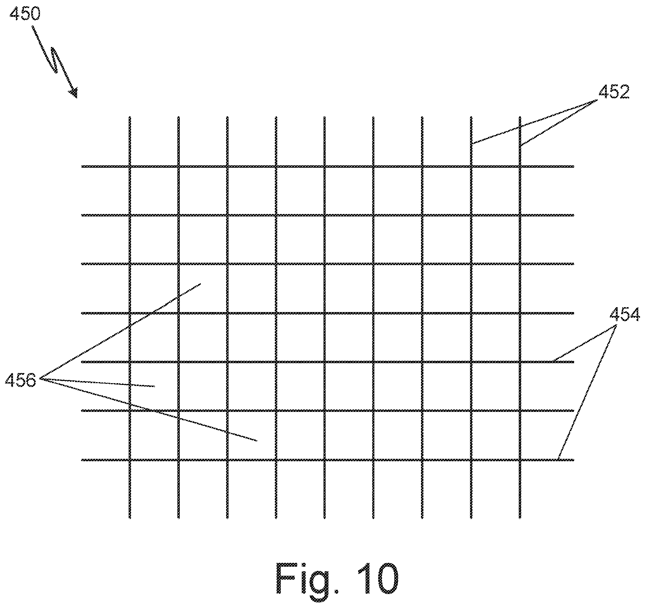

FIG. 9A includes PET yarn 422 and ferromagnetic yarn 424, which can be combined using techniques discussed in FIGS. 10-13 to create ferromagnetic fabrics. PET yarn 422 is made from polyethylene terephthalate (PET), a biocompatible plastic material. PET yarn 422 starts as PET chips, which are melted or extruded into fibers. Multiple extruded fibers can be used to mold PET yarn 422. Similar biocompatible plastic yarns can be made from other biocompatible plastics using a similar process as described for creating PET yarn.

FIG. 9A also includes ferromagnetic yarn 424. Creating ferromagnetic yarn 424 depends on the specific form ferromagnetic materials take. Ferrite material occurring as nanoparticles will need to undergo electrospinning to be synthesized into ferromagnetic fibers, which can be used alone or spun together to create ferromagnetic yarn 424. Ferromagnetic fibers are approximately 0.2-0.3 millimeters thick when used as ferromagnetic yarn 424 in a fabric (for example, fabric 400 of FIG. 7 or fabric 410 of FIG. 8). Alternatively, if ferrite materials occur as fibers, such fibers can be used singularly or combined to become ferromagnetic yarn 424. Example ferrite materials include zinc ferrite and manganese ferrite.

FIG. 9B shows combined yarn 430, which includes PET fiber 432 and ferromagnetic fiber 434. PET fiber 432 and ferromagnetic fiber 434 are synthesized as discussed in relation to FIG. 9A. PET fiber 432 and ferromagnetic fiber 434 can then be combined (for example, by spinning) and used as a single yarn used to create fabrics for use as skirt 280 and skirt 380. Combined yarn 430 can also include radiopaque fibers in some examples.

FIG. 9C shows coated yarn 440, which includes PET yarn 442 and ferromagnetic coating 444. PET yarn 442 can be made by processes described in relation to FIG. 9A. Ferromagnetic coating 444 can then be applied to an outer surface of PET yarn 442. Radiopaque fibers can be included in coated yarn 440 by spinning PET yarn 442 with radiopaque fibers before coating with ferromagnetic coating 444. Alternatively, ferromagnetic coating 444 can include radiopaque materials.

FIG. 10 is a schematic view of woven fabric 450. Woven fabric 450 includes warp yarn 452, weft yarn 454, and pores 456. Woven fabric 450 can be used to form skirt 280 of FIGS. 3-5 or skirt 380 of FIG. 6. Woven fabric 450 is created by interlacing yarn (for example, PET yarn 422, ferromagnetic yarn 424, combined yarn 430, and/or coated yarn 440 in FIGS. 9A-9C). Warp yarn 452 is stretched onto a loom and weft yarn 454 is woven between warp yarn 452. Pores 456 are holes extending through woven fabric 450 between warp yarn 452 and weft yarn 454. Pores 456 of woven fabric 450 are between 0.5 micrometers and 1000 micrometers. In woven fabric 450, pores 456 are uniformly sized, distributed, and connected. Woven fabric 450 is a sturdy, non-stretchy fabric.

Woven fabric 450 can be created through many different combinations of yarns as warp yarn 452 and weft yarn 454. In a first example, warp yarn 452 and weft yarn 454 are both made of PET yarn (for example, PET yarn 422 in FIG. 9A) to create a PET woven fabric 450. The PET woven fabric 450 can be used as PET fabric 402 in fabric 400 (shown in FIG. 7) or PET fabric 412 in fabric 410 (shown in FIG. 8). In a second example, warp yarn 452 and weft yarn 454 are both made of ferromagnetic yarn (for example, ferromagnetic yarn 424 of FIG. 9A, combined yarn 430 of FIG. 9B, or coated yarn 440 of FIG. 9C) to create a ferromagnetic woven fabric 450. The ferromagnetic woven fabric 450 can be used as ferromagnetic fabric 414 in fabric 410 or be used directly to create skirt 280 (shown in FIGS. 3-5) or ferromagnetic skirt 380 (shown in FIG. 6). Radiopaque fibers can also be integrated into ferromagnetic woven fabric 450 in some examples.

Combinations of ferromagnetic yarn and PET yarn can also be used as warp yarn 452 and weft yarn 454. Radiopaque fibers or yarn can also be integrated into weaving fabric 450 in some examples, as necessary. In a third example, warp yarn 452 is ferromagnetic yarn and weft yarn is PET yarn. Alternatively, warp yarn 452 is PET yarn and weft yarn 454 is ferromagnetic yarn. In this example, a combined fabric 450 is made that can be used to fashion skirt 280 and ferromagnetic skirt 380. In a fourth example, various combinations of PET yarn and ferromagnetic yarn can be used as warp yarn 452 and weft yarn 454 to create a patterned ferromagnetic fabric 450. In this example, every other warp yarn 452 and every other weft yarn 454 could be a ferromagnetic yarn and the balance of yarns 452-454 are PET yarn. Any pattern of alternating ferromagnetic yarn as warp yarn 452 and weft yarn 454 with the balance being PET yarn can be used to create the patterned ferromagnetic fabric 450. The pattern of the patterned ferromagnetic fabric 450 can also be used to fashion skirt 280 and ferromagnetic skirt 380. Changing the pattern of patterned ferromagnetic woven fabric 450 changes the density of ferromagnetic (and radiopaque) material in the final product.

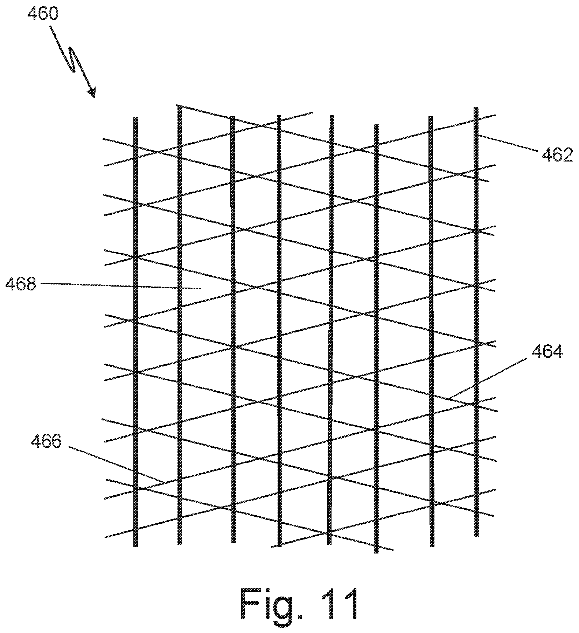

FIG. 11 is a schematic view of braided fabric 460, which includes axial tows 462, first braider tows 464, second braider tows 466, and pores 468. Braided fabric 460 can be used to form skirt 280 shown in FIGS. 3-5 or ferromagnetic skirt 380 shown in FIG. 6. Braided fabric 460 is created with three yarns. Braiding can alternatively be done with two yarns or four yarns. Braiding can also be used to create sutures (for example sutures 272 in FIG. 3). Axial tows 462 are a first yarn and are stretched onto a loom. First braider tows 464 are a second yarn and second braider tows 466 are a third yarn. First braider tows 464 and second braider tows 466 are intertwined through axial tows 462 and each other to create braided fabric 460. First braider tows 464 are offset between 30° and 60° in a first direction from axial tows 462. Second braider tows 466 are offset between 30° to 60° in a second direction from axial tows 462. The second direction is opposite from the first direction. Dimensions of braided fabric 460 depends on the number of axial tows 462 used. Pores 468 are holes extending through braided fabric 460 between axial tows 462, first braider tows 464, and second braider tows 466. Pores 468 in braided fabric 460 are between 0.5 micrometers and 1000 micrometers. Braided fabric 460 has uniform pore size, distribution, and connectivity. Braided fabric 460 will have some give in the radial direction (perpendicular to axial tows 462). However, braided fabric 460 is sturdy in the axial direction. Braided fabric 460 can be flat or tube-shaped.

Braided fabric 460 can be created using different combinations of yarn types. In a first example, axial tows 462, first braider tows 464, and second braider tows 466 are all made of PET yarn (for example, PET yarn 422 in FIG. 9A) to create a PET braided fabric 460. The PET braided fabric 460 can be used as PET fabric 402 in fabric 400 (shown in FIG. 7) or PET fabric 412 in fabric 410 (shown in FIG. 8). In a second example, axial tows 462, first braider tows 464, and second braider tows 466 are all made of ferromagnetic yarn (for example, ferromagnetic yarn 424 of FIG. 9A, combined yarn 430 of FIG. 9B, or coated yarn 440 of FIG. 9C) to create a ferromagnetic braided fabric 460. The ferromagnetic braided fabric 460 can be used as ferromagnetic fabric 414 in fabric 410 or be used alone to create skirt 280 (shown in FIGS. 3-5) or ferromagnetic skirt 380 (shown in FIG. 6). A radiopaque yarn can also be used as axial tows 462, first braider tows 464, or second braider tows 468 when creating the ferromagnetic braided fabric 460.

Combinations of ferromagnetic yarn and PET yarn can also be used as axial tows 462, first braider tows 464, and second braider tows 466. In a third example, axial tows 462 are ferromagnetic yarn and first braider tows 464 and second braider tows 466 are PET yarn. Alternatively, axial tows 462 can be PET yarn and first braider tows 464, and second braider tows 466 can be ferromagnetic yarn. In this example, a combined braided fabric 460 is made that can be used to fashion skirt 280 or ferromagnetic skirt 380 (shown in FIGS. 3-6). In a fourth example, various combinations of PET yarn and ferromagnetic yarn can be used as axial tows 462, first braider tows 464, and second braider tows 466 to create a patterned braided fabric 460. In this example, every other axial tow 462, first braider tow 464, and second braider tow 466 could be a ferromagnetic yarn with the balance being made of PET yarn. Any pattern of alternating ferromagnetic yarn and PET can be used to create the patterned ferromagnetic fabric. The patterned braided fabric 460 can also be used to fashion skirt 280 or ferromagnetic skirt 380.

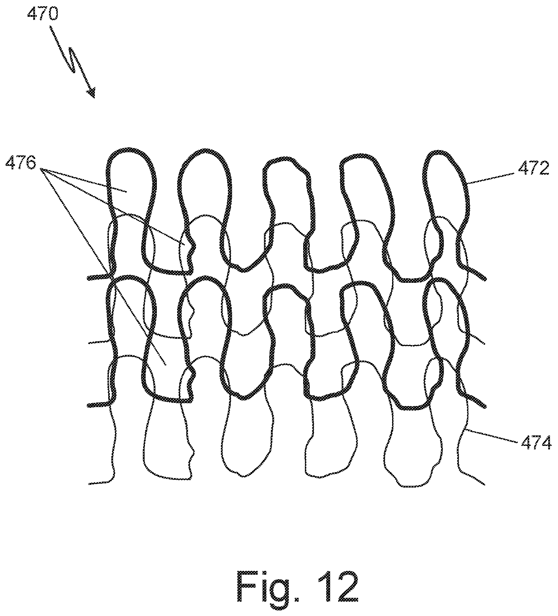

FIG. 12 is a schematic view of knit fabric 470, which includes first yarn 472, second yarn 474, and pores 476. Knit fabric 470 can be used to fashion skirt 280 or ferromagnetic skirt 380 (shown in FIGS. 3-6). First yarn 472 and second yarn 474 are interlooped with one another to create knit fabric 470. Knit fabric 470 shows rows of knitting, or interlooped yarns, with a top row and a third row (starting from a top of FIG. 12) made of first yarn 472 and a second row and a fourth row made of second yarn 474. First yarn 472 and second yarn 474 are differentiated in FIG. 12 to illustrate the rows and how different types of yarn can be combined. However, first yarn 472 and second yarn 474 can alternatively be the same type or strand of yarn (or yarns) which is continuously knit to form knit fabric 470. Pores 476 are holes extending through knit fabric 470 between first yarn 472 and second yarn 474. Pores 476 in knit fabric 470 are between 50 micrometers and 1000 micrometers. Pores 476 are irregularly distributed, sized, and connected in knit fabric 470. Knit fabric 470 can stretch in multiple directions including a parallel direction to the yarn and a perpendicular direction to the yarn. As such, knit fabric 470 is flexible and pore sizes 476 vary depending on tension applied to knit fabric 470.

Knit fabric 470 can be created using different combinations of yarn types. In a first example, first yarn 472 and second yarn 474 are PET yarn (for example, PET yarn 422 in FIG. 9A), which creates a PET knit fabric 470. The PET knit fabric 470 can be used as PET fabric 402 in fabric 400 (shown in FIG. 7) or PET fabric 412 in fabric 410 (shown in FIG. 8). In a second example, first yarn 472 and second yarn 474 are ferromagnetic yarn (for example, ferromagnetic yarn 424 of FIG. 9A, combined yarn 430 of FIG. 9B, or coated yarn 440 of FIG. 9C), which creates a ferromagnetic knit fabric 470. The ferromagnetic knit fabric 470 can be used as ferromagnetic fabric 414 in fabric 410 or be used alone to create skirt 280 or ferromagnetic skirt 380 (shown in FIGS. 3-6). Combinations of ferromagnetic yarn and PET yarn can be used to create a patterned knit fabric 470. Any pattern can be used to create knit fabric 470. In a third example, a striped pattern can be created using PET yarn as first yarn 472 and ferromagnetic yarn as second yarn 474. Alternatively, first yarn 472 can be PET yarn and second yarn 474 can be ferromagnetic yarn. In a fourth example, he patterned knit fabric 470 can also be created by alternating the type of yarn used on individual loops of each row. This would create a vertical striping pattern or checkerboard pattern. Additional knitting patterns are contemplated herein. The patterned knit fabric 470 can be used to fashion skirt 280 and ferromagnetic skirt 380.

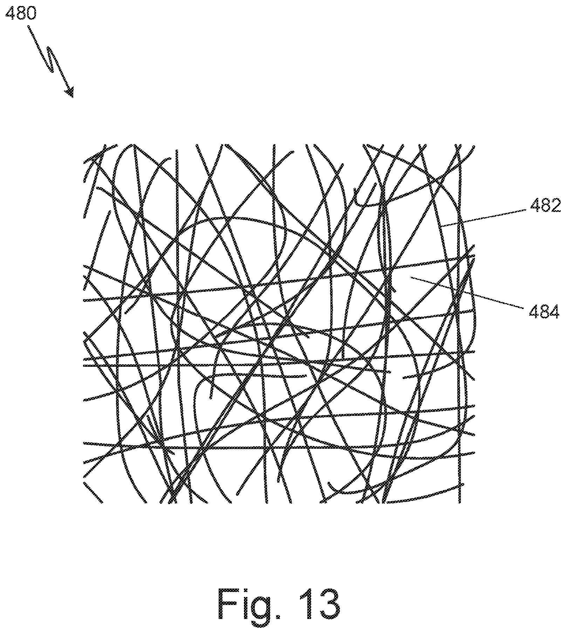

FIG. 13 is a schematic view of non-woven fabric 480, which includes yarns 482 and pores 484. Non-woven fabric 480 can be used to form ferromagnetic skirt 280 of FIGS. 3-5 or ferromagnetic skirt 380 of FIG. 6. Non-woven fabric 480 is made of randomly oriented yarns 482. Yarns 482 can be PET yarn, ferromagnetic yarn, radiopaque yarn, and combinations thereof. Example yarns that can be used as yarn 482 to make non-woven fabric 480 are discussed in relation to FIGS. 9A-9C. Non-woven fabric 480 can be created using an electrospinning process. Other methods of creating non-woven fabric (for example, spun-bonding and melt-blowing) can also be used to create non-woven fabric 480. Pores 484 are holes extending through knit fabric 480 yarns 482. Pores 484 in non-woven fabric 480 are between 10 micrometers and 1000 micrometers. In non-woven fabric 480, pores 484 have variable sizing, distribution, and connectivity due to the random orientation of yarns 482.

Non-woven fabric 480 can be created using different combinations of yarn types. In a first example, yarn 482 is PET yarn (for example, PET yarn 422 in FIG. 9A), which creates a PET non-woven fabric 480. The PET non-woven fabric 480 can be used as PET fabric 402 in fabric 400 (shown in FIG. 7) or PET fabric 412 in fabric 410 (shown in FIG. 8). In a second example, yarn 482 is ferromagnetic yarn (for example, ferromagnetic yarn 424 of FIG. 9A, combined yarn 430 of FIG. 9B, or coated yarn 440 of FIG. 9C), which creates a ferromagnetic non-woven fabric 480. The ferromagnetic non-woven fabric 480 can be used as ferromagnetic fabric 414 in fabric 410 or be used alone to fashion skirt 280 or ferromagnetic skirt 380 (shown in FIGS. 3-6).

Combinations of ferromagnetic yarn and PET yarn can be used to create a combined non-woven fabric. In a third example, a combination of PET yarn and ferromagnetic yarn can used simultaneously as yarn 482. This creates a combined non-woven fabric 480 which can be used to fashion skirt 280 or ferromagnetic skirt 380. In a fourth example, PET yarn and ferromagnetic yarn can be used in an alternating pattern to create layers. In this example, ferromagnetic yarn is used as yarn 482 to create a first layer, then PET yarn is used to create a second layer. The resulting non-woven fabric 480 would have a structure like fabric 410. This example also includes an additional layer of ferromagnetic yarn creating a third layer. Using this method, fabric 480 could be used as fabric 400 with the first and third layer being ferromagnetic layers 414 and the second layer being PET layer 412. Thicknesses of different layers can be varied depending on properties desired in non-woven fabric 480. Radiopaque yarn can also be combined into non-woven fabric 480.

Any of the various systems, devices, apparatuses, etc. in this disclosure can be sterilized (e.g., with heat, radiation, ethylene oxide, hydrogen peroxide, etc.) to ensure they are safe for use with patients, and the methods herein can comprise sterilization of the associated system, device, apparatus, etc. (e.g., with heat, radiation, ethylene oxide, hydrogen peroxide, etc.).

The treatment techniques, methods, steps, etc. described or suggested herein or in references incorporated herein can be performed on a living animal or on a non-living simulation, such as on a cadaver, cadaver heart, anthropomorphic ghost, simulator (e.g., with the body parts, tissue, etc. being simulated), etc.

While the invention has been described with reference to an exemplary embodiment(s), it will be understood by those skilled in the art that various changes may be made without departing from the scope of the invention. In addition, many modifications may be made to adapt a particular situation or material to the teachings of the invention without departing from the essential scope thereof. Therefore, it is intended that the invention not be limited to the particular embodiment(s) disclosed, but that the invention will include all embodiments falling within the scope of the appended claims.

## Claims

1. An implantable medical device comprising:
a wire frame (246) formed of struts (258) and openings (260);
a cover (280) connected to the struts of the wire frame, wherein the cover is fashioned from a fabric (400), the fabric comprising:
a PET fabric (402) made of PET yarn, wherein the PET yarn is made from a plurality of PET fibers; and
a ferromagnetic material (404) combined with the PET fabric; and
a sensor (226) positioned on the cover and connected to the wire frame;
wherein the cover is configured to shield the sensor from detuning effects of the wire frame.

2. The implantable medical device of claim 1, wherein the PET fabric and the ferromagnetic material are both biocompatible materials, and/or wherein the ferromagnetic material has a high radiopacity.

3. The implantable medical device of claim 1 or claim 2, wherein the ferromagnetic material is chosen from the group consisting of iron, manganese, zinc, and combinations thereof.

4. The implantable medical device of any preceding claim, wherein the cover is flexible and configured to crimp with the wire frame when the wire frame is in a crimped state and expand with the wire frame as the wire frame moves from the crimped state to an expanded state.

5. The implantable medical device of any preceding claim, wherein the ferromagnetic material is a coating of ferromagnetic material on the PET fabric.

6. The implantable medical device of claim 5, wherein the coating of ferromagnetic material is:
a) on both sides of the PET fabric; or
b) adjacent to the sensor.

7. The implantable medical device of any preceding claim, wherein the ferromagnetic material is a ferromagnetic fabric formed of a ferromagnetic yarn, and wherein the ferromagnetic yarn is formed of a plurality of ferromagnetic fibers.

8. The implantable medical device of claim 7, wherein the ferromagnetic fibers are formed by electrospinning ferromagnetic nanoparticles into the ferromagnetic fibers.

9. The implantable medical device of claim 7 or claim 8, wherein the cover includes the PET fabric stitched to the ferromagnetic fabric, and wherein a set of stitching holds the PET fabric, the ferromagnetic fabric, and the wire frame together.

10. The implantable medical device of claim 7 or claim 8, wherein the PET fabric is adhered to the ferromagnetic fabric.

11. The implantable medical device of any of claims 7 to 10, wherein:
a) the ferromagnetic fabric is adjacent to the sensor; and/or
b) the ferromagnetic fabric is a woven ferromagnetic fabric formed of interlacing the ferromagnetic yarn;
the ferromagnetic fabric is a knit ferromagnetic fabric formed of interlooping the ferromagnetic yarn;
the ferromagnetic fabric is a braided fabric formed of intertwining the ferromagnetic yarn; or
the ferromagnetic fabric is a non-woven fabric formed by electrospinning the ferromagnetic yarn.

12. The implantable medical device of any of claims 1 to 6, wherein the ferromagnetic material is a plurality of ferromagnetic fibers, and wherein:
a) the plurality of ferromagnetic fibers are woven with the plurality of PET fibers to create the fabric of the cover; or
b) the plurality of ferromagnetic fibers and the plurality of PET fibers are knit together to create the fabric of the cover.

13. The implantable medical device of any preceding claim, wherein the cover:
a) is connected to the struts of the wire frame by stitching the cover onto the struts, and wherein the sensor is connected to the wire frame by stitching the sensor onto the wire frame, and wherein the stitching extends through the cover; and/or
b) extends from a top of the wire frame to a bottom of the wire frame.

14. The implantable medical device of any of claims 1 to 4, wherein the ferromagnetic material is a coating of ferromagnetic material on the plurality of PET fibers,
optionally wherein:
the plurality of PET fibers with the ferromagnetic material coating are woven together to form the fabric;
the plurality of PET fibers with the ferromagnetic material coating are knit together to form the fabric; or
the ferromagnetic material is a plurality of ferromagnetic fibers coating the PET fibers by being braided around the PET fibers.

15. The implantable medical device of any preceding claim, wherein the device is sterilized.

## Patentansprüche

1. Implantierbare medizinische Vorrichtung umfassend:
einen Drahtrahmen (246), der aus Streben (258) und Öffnungen (260) gebildet ist;
eine Abdeckung (280), die mit den Streben des Drahtrahmens verbunden ist, wobei die Abdeckung aus einem Gewebe (400) gebildet ist, wobei das Gewebe umfasst:
ein PET-Gewebe (402) aus PET-Garn, wobei das PET-Garn aus einer Mehrzahl von PET-Fasern besteht; und
ein ferromagnetisches Material (404) kombiniert mit dem PET-Gewebe; und
einen Sensor (226), der auf der Abdeckung angeordnet und mit dem Drahtrahmen verbunden ist;
wobei die Abdeckung dazu ausgelegt ist, den Sensor vor Verstimmungseffekten des Drahtrahmens abzuschirmen.

2. Implantierbare medizinische Vorrichtung nach Anspruch 1, wobei das PET-Gewebe und das ferromagnetische Material beide biokompatible Materialien sind und/oder wobei das ferromagnetische Material eine hohe Röntgensichtbarkeit aufweist.

3. Implantierbare medizinische Vorrichtung nach Anspruch 1 oder Anspruch 2, wobei das ferromagnetische Material ausgewählt ist aus der Gruppe bestehend aus Eisen, Mangan, Zink und Kombinationen davon.

4. Implantierbare medizinische Vorrichtung nach einem der vorstehenden Ansprüche, wobei die Abdeckung flexibel und dazu ausgelegt ist, sich mit dem Drahtrahmen zu falten, wenn der Drahtrahmen in einem gefalteten Zustand vorliegt, und mit dem Drahtrahmen zu expandieren, wenn sich der Drahtrahmen von dem gefalteten Zustand in einen expandierten Zustand bewegt.

5. Implantierbare medizinische Vorrichtung nach einem der vorstehenden Ansprüche, wobei das ferromagnetische Material eine Beschichtung aus ferromagnetischem Material auf dem PET-Gewebe ist.

6. Implantierbare medizinische Vorrichtung nach Anspruch 5, wobei die Beschichtung aus ferromagnetischem Material vorliegt:
a) an beiden Seiten des PET-Gewebes; oder
b) benachbart zu dem Sensor.

7. Implantierbare medizinische Vorrichtung nach einem der vorstehenden Ansprüche, wobei das ferromagnetische Material ein ferromagnetisches Gewebe ist, das aus einem ferromagnetischen Garn gebildet ist, und wobei das ferromagnetische Garn aus einer Mehrzahl von ferromagnetischen Fasern gebildet ist.

8. Implantierbare medizinische Vorrichtung nach Anspruch 7, wobei die ferromagnetischen Fasern durch Elektrospinnen von ferromagnetischen Nanopartikeln in die ferromagnetischen Fasern gebildet werden.

9. Implantierbare medizinische Vorrichtung nach Anspruch 7 oder Anspruch 8, wobei die Abdeckung das PET-Gewebe enthält, das an das ferromagnetischen Gewebe genäht ist, und wobei ein Satz von Nähten das PET-Gewebe, das ferromagnetische Gewebe und den Drahtrahmen zusammenhält.

10. Implantierbare medizinische Vorrichtung nach Anspruch 7 oder Anspruch 8, wobei das PET-Gewebe an das ferromagnetische Gewebe gehaftet ist.

11. Implantierbare medizinische Vorrichtung nach einem der Ansprüche 7 bis 10, wobei:
a) das ferromagnetische Gewebe benachbart zu dem Sensor liegt; und/oder
b) das ferromagnetische Gewebe ein gewebtes ferromagnetisches Gewebe ist, das durch Verflechten des ferromagnetischen Garns gebildet ist;
das ferromagnetische Gewebe ein gewirktes ferromagnetisches Gewebe ist, das durch Verschlingen des ferromagnetischen Garns gebildet ist;
das ferromagnetische Gewebe ein geflochtenes Gewebe ist, das durch Verflechten des ferromagnetischen Garns gebildet ist; oder
das ferromagnetische Gewebe Vlies ist, das durch Elektrospinnen des ferromagnetischen Garns gebildet ist.

12. Implantierbare medizinische Vorrichtung nach einem der Ansprüche 1 bis 6, wobei das ferromagnetische Material eine Mehrzahl von ferromagnetische Fasern ist und wobei:
a) die Mehrzahl von ferromagnetischen Fasern mit der Mehrzahl von PET-Fasern verwebt ist, um das Gewebe der Abdeckung zu erzeugen; oder
b) die Mehrzahl von ferromagnetischen Fasern und die Mehrzahl von PET-Fasern miteinander verstrickt sind, um das Gewebe der Abdeckung zu erzeugen.

13. Implantierbare medizinische Vorrichtung nach einem der vorstehenden Ansprüche, wobei die Abdeckung:
a) mit den Streben des Drahtrahmens durch Nähen der Abdeckung an die Streben verbunden ist, und wobei der Sensor durch Nähen des Sensors an den Drahtrahmen mit dem Drahtrahmen verbunden ist und wobei die Naht durch die Abdeckung verläuft; und/oder
b) von einer Oberseite des Drahtrahmens zu einer Unterseite des Drahtrahmens verläuft.

14. Implantierbare medizinische Vorrichtung nach einem der Ansprüche 1 bis 4, wobei das ferromagnetische Material eine Beschichtung aus ferromagnetischem Material auf der Mehrzahl von PET-Fasern ist,
gegebenenfalls wobei:
die Mehrzahl von PET-Fasern mit der Beschichtung aus ferromagnetischem Material miteinander verwoben ist, um das Gewebe zu bilden;
die Mehrzahl von PET-Fasern mit der Beschichtung aus ferromagnetischem Material miteinander verstrickt sind, um das Gewebe zu bilden; oder
das ferromagnetische Material eine Mehrzahl von ferromagnetischen Fasern ist, die die PET-Fasern durch Umflechten um die PET-Fasern beschichten.

15. Implantierbare medizinische Vorrichtung nach einem der vorstehenden Ansprüche, wobei die Vorrichtung sterilisiert ist.

## Revendications

1. Dispositif médical implantable comprenant :
un cadre métallique (246) formé d'entretoises (258) et d'ouvertures (260) ;
une couverture (280) reliée aux entretoises du cadre métallique, la couverture étant confectionnée à partir d'un tissu (400), le tissu comprenant :
un tissu de PET (402) composé de fil de PET, dans lequel le fil de PET est composé d'une pluralité de fibres de PET ; et
un matériau ferromagnétique (404) combiné avec le tissu de PET ; et
un capteur (226) positionné sur la couverture et relié au cadre métallique,
dans lequel la couverture est configurée pour protéger le capteur contre des effets de désaccord du cadre métallique.

2. Dispositif médical implantable selon la revendication 1, dans lequel le tissu de PET et le matériau ferromagnétique sont tous deux des matériaux biocompatibles, et/ou dans lequel le matériau ferromagnétique a une opacité élevée.

3. Dispositif médical implantable selon la revendication 1 ou la revendication 2, dans lequel le matériau ferromagnétique est choisi dans le groupe constitué par le fer, le manganèse, le zinc et leurs combinaisons.

4. Dispositif médical implantable selon l'une quelconque des revendications précédentes, dans lequel la couverture est flexible et configuré pour se sertir avec le cadre métallique lorsque le cadre métallique est dans un état serti et se dilater avec le cadre métallique lorsque le cadre métallique passe de l'état serti à un état dilaté.

5. Dispositif médical implantable selon l'une quelconque des revendications précédentes, dans lequel le matériau ferromagnétique est un revêtement de matériau ferromagnétique sur le tissu de PET.

6. Dispositif médical implantable selon la revendication 5, dans lequel le revêtement de matériau ferromagnétique est :
a) sur les deux côtés du tissu de PET ; ou
b) adjacent au capteur.

7. Dispositif médical implantable selon l'une quelconque des revendications précédentes, dans lequel le matériau ferromagnétique est un tissu ferromagnétique formé d'un fil ferromagnétique, et dans lequel le fil ferromagnétique est formé d'une pluralité de fibres ferromagnétiques.

8. Dispositif médical implantable selon la revendication 7, dans lequel les fibres ferromagnétiques sont formées par électrofilage de nanoparticules ferromagnétiques dans les fibres ferromagnétiques.

9. Dispositif médical implantable selon la revendication 7 ou la revendication 8, dans lequel la couverture comprend le tissu de PET cousu sur le tissu ferromagnétique, et dans lequel un ensemble de coutures maintient ensemble le tissu de PET, le tissu ferromagnétique et le cadre métallique.

10. Dispositif médical implantable selon la revendication 7 ou la revendication 8, dans lequel le tissu de PET est collé au tissu ferromagnétique.

11. Dispositif médical implantable selon l'une quelconque des revendications 7 à 10, dans lequel :
a) le tissu ferromagnétique est adjacent au capteur ; et/ou
b) le tissu ferromagnétique est un tissu ferromagnétique formé en entrelaçant le fil ferromagnétique ;
le tissu ferromagnétique est un tissu ferromagnétique tricoté formé par imbrication de boucles du fil ferromagnétique ;
le tissu ferromagnétique est un tissu tressé formé d'entrecroisement du fil ferromagnétique ; ou
le tissu ferromagnétique est un tissu non tissé formé par électrofilage du fil ferromagnétique.

12. Dispositif médical implantable selon l'une quelconque des revendications 1 à 6, dans lequel le matériau ferromagnétique est une pluralité de fibres ferromagnétiques, et dans lequel :
a) la pluralité de fibres ferromagnétiques sont tissées avec la pluralité de fibres de PET pour créer le tissu de la couverture ; ou
b) la pluralité de fibres ferromagnétiques et la pluralité de fibres de PET sont tricotées ensemble pour créer le tissu de la couverture.

13. Dispositif médical implantable selon l'une quelconque des revendications précédentes, dans lequel la couverture :
a) est reliée aux entretoises du cadre métallique par couture de la couverture sur les entretoises, et dans lequel le capteur est relié au cadre métallique par couture du capteur sur le cadre métallique, et dans lequel la couture s'étend à travers la couverture ; et/ou
b) s'étend d'une partie supérieure du cadre métallique à une partie inférieure du cadre métallique.

14. Dispositif médical implantable selon l'une quelconque des revendications 1 à 4, dans lequel le matériau ferromagnétique est un revêtement de matériau ferromagnétique sur la pluralité de fibres de PET,
éventuellement dans lequel :
la pluralité de fibres de PET avec le revêtement de matériau ferromagnétique sont tissés ensemble pour former le tissu ;
la pluralité de fibres de PET avec le revêtement de matériau ferromagnétique sont tricotés ensemble pour former le tissu ; ou
le matériau ferromagnétique est une pluralité de fibres ferromagnétiques revêtant les fibres de PET en étant tressées autour des fibres de PET.

15. Dispositif médical implantable selon l'une quelconque des revendications précédentes, dans lequel le dispositif est stérilisé.
